(19) Europäisches Patentamt
European Patent Office
Office européen des brevets

(11) Numéro de publication : **0 109 340**
**B1**

(12) # FASCICULE DE BREVET EUROPÉEN

(45) Date de publication du fascicule du brevet : **16.04.86**

(21) Numéro de dépôt : **83402168.5**

(22) Date de dépôt : **09.11.83**

(51) Int. Cl.⁴ : **C 07 D239/42**, A 61 K 31/505, C 07 C 55/02, C 07 C 57/15, C 07 C 57/145, C 07 C 59/245, C 07 C101/20, C 07 C101/22

(54) **Sels acides de 2-pipérazinopyrimidine, procédé pour leur préparation et compositions pharmaceutiques en contenant.**

(30) Priorité : **12.11.82 FR 8218975**

(43) Date de publication de la demande : **23.05.84 Bulletin 84/21**

(45) Mention de la délivrance du brevet : **16.04.86 Bulletin 86/16**

(84) Etats contractants désignés : **AT BE CH DE FR GB IT LI LU NL SE**

(56) Documents cités :
**US-A- 2 985 657**
**JOURNAL OF CHROMATOGRAPHY, vol. 252, 3 décembre 1982, pages 310-314, Elsevier Scientific Publishing Company S. CACCIA et al.: "Identification and quantitation of 1-(2-pyrimidinyl)piperazine, an active metabolite of the anxiolytic agent buspirone, in rat plasma and brain"**

(73) Titulaire : **SANOFI, société anonyme**
**40, Avenue George V**
**F-75008 Paris (FR)**

(72) Inventeur : **Crisafulli, Emilio**
**Viale San Gimignano 12**
**I-20146 Milan (IT)**
Inventeur : **Frigerio, Marco**
**Via Carlo Poma 28**
**Mantova (IT)**
Inventeur : **Chambon, Jean-Pierre**
**Chemin des Truquets Route de Montpellier**
**F-34570 Montarnaud (FR)**

(74) Mandataire : **Gillard, Marie-Louise et al**
**Cabinet Beau de Loménie 55, Rue d'Amsterdam**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 109 340 B1**

Jouve, 18, rue St-Denis, 75001 Paris, France

**Description**

La présente invention concerne de nouveaux sels acides de 2-pipérazinopyrimidine ayant une action psychotrope dopaminergique.

Plus particulièrement, l'invention se réfère à des sels acides de 2-(1-pipérazinyl)pyrimidine avec des acides dicarboxyliques, à un procédé pour leur préparation et à des compositions pharmaceutiques les renfermant en tant que principes actifs.

La 2-(1-pipérazinyl)pyrimidine est, en tant que base libre, un composé bien connu en littérature qui est utilisé en tant qu'intermédiaire de synthèse.

Il a été testé parmi toute une série de composés dont aucun n'a montré d'activté analgésique ou antifilariale (H.W. Stewart et al., J. Org. Chem. 1953, 18, 1478).

Son sel d'addition avec l'acide chlorhydrique est décrit dans le brevet indien 147 985 comme intermédiaire dans la synthèse de dérivés de la tétracycline.

On a maintenant trouvé que les sels acides de la 2-(1-pipérazinyl)pyrimidine avec les acides dicarboxyliques possèdent une très bonne activté psychotrope avec un mécanisme d'action dopaminergique, notamment une activité antipsychotique, antidépressive et tranquillisante-sédative.

Ainsi la présente invention a pour objet, selon un de ses aspects, les sels acides de la 2-(1-pipérazinyl)pyrimidine avec des acides dicarboxyliques.

Les sels acides préférés de la 2-(1-pipérazinyl)pyrimidine avec des acides dicarboxyliques sont représentés par la formule :

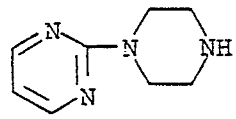 , HOOC-X-COOH

dans laquelle X représente un groupe alkylène ayant 1 à 3 atomes de carbone, un groupe vinylène, un groupe hydroxyéthylène un groupe 1,2-dihydroxyéthylène, un groupe aminoéthylène ou un groupe amino-1,3-propylène.

Particulièrement préférés sont le mono-L-(+)-tartrate (formule I, X = 1,2-dihydroxyéthylène), le monosuccinate (formule I, X = éthylène), le monomaléate (formule I, X = vinylène, cis-), le monofumarate (formule I, X = vinylène, trans) et le monoglutamate (formule I, X = 3-amino-1,3-propylène) de la 2-(1-pipérazinyl)pyrimidine.

Les composés de la présente invention sont préparés, selon un autre aspect de la présente invention, par un procédé de salification caractérisé en ce qu'on traite la 2-(1-pipérazinyl)pyrimidine avec la quantité calculée de l'acide dicarboxylique dans un solvant organique ou organique aqueux.

Comme acide dicarboxylique, on utilise de préférence, en quantité équimoléculaire, un composé de formule

HOOC—X—COOH

dans laquelle X est tel que défini ci-dessus, les acides L(+)-tartrique, succinique, maléique, fumarique et L-glutamique étant particulièrement préférés.

L'acide dicarboxylique peut être utilisé tel quel ou bien dissous dans le même solvant utilisé pour la 2-(1-pipérazinyl)pyrimidine ou encore dissous dans l'eau.

Comme solvant organique on utilise un alcool, l'isopropanol de préférence, l'acétone, l'acétate d'éthyle ou similaires.

Le sel ainsi obtenu est isolé selon les techniques conventionnelles par simple filtration et cristallisation éventuelle.

La salification peut être conduite sur une 2-(1-pipérazinyl)pyrimidine à l'état brut telle qu'obtenue de la réaction entre la 2-chloropyrimidine et la pipérazine selon des modes opératoires connus.

L'activité des composés de la présente invention a été évaluée dans les tests prédictifs des activités antipsychotique, antidépressive et tranquillisante-sédative et du mécanisme d'action dopaminergique.

L'activité antipsychotique d'un composé représentatif, le monomaléate de 2-(1-pipérazinyl)pyrimidine, a été déterminée dans le test de l'antagonisme de la toxicité de groupe due à l'amphétamine (J. H. Burn et al., Arch. Int. Pharmacodyn. Ther. 1958, 113, 290).

Le monomaléate de 2-(1-pipérazinyl)pyrimidine a été administré à des doses croissantes par voie intrapéritonéale à des lots de 10 souris Charles Fiver CD1 pesant 20-22 g et groupées dans des cages de 22 cm de long, 12 cm de large et 12 cm de haut. Trente minutes après l'administration, on a injecté par voie intrapéritonéale de l'amphétamine à la dose de 30 mg/kg. La mortalité des animaux a été évaluée durant les 24 h qui ont suivi l'injection d'amphétamine et comparée, dans chaque groupe, à celle du groupe d'animaux témoins n'ayant reçu que le véhicule (sérum physiologique).

Le tableau ci-dessous montre la dose efficace médiane (DE50) d'antagonisme de la mortalité due à l'amphétamine.

Tableau I

| Composé | DE50 (limites de confiance) |
| --- | --- |
| 2-(1-pipérazinyl)pyrimidine monomaléate | 16,5 mg/kg (10,1 - 26,7) |

De ce tableau, il ressort que le composé de la présente invention possède une bonne activité dans le test de la toxicité de groupe due à l'amphétamine, prédictif d'une action antipsychotique.

L'activité antidépressive des composés de l'invention a été évaluée dans le test de l'antagonisme vis-à-vis de l'installation de la catalepsie provoquée par la prochlorpérazine (K. Bizière et al., Arzneimittel Forschung, 1982, 32 (II), 824).

Un composé représentatif de l'invention, à savoir le monomaléate de 2-(1-pipérazinyl)pyrimidine, a été administré par voie intrapéritonéale à des groupes de 10 rats mâles Wistar pesant 220-240 g ; des animaux de contrôle ont été en même temps traités par du sérum physiologique. Une heure après, les animaux ont reçu, par voie sous-cutanée, de la prochlorpérazine à une dose de 10 mg/kg. Cinq heures après cette administration, le nombre d'animaux cataleptiques a été estimé par le test du bouchon. Ce test consiste à positionner les animaux de telle sorte qu'ils puissent maintenir pendant au moins 20 secondes leurs pattes en appui sur une colonne formée de trois bouchons de liège superposés (hauteur totale 11 cm). Les performances réalisées pour chaque groupe d'animaux ont été comparées à celles du groupe d'animaux témoins n'ayant reçu que le véhicule et la prochlorpérazine.

Le tableau ci-dessous montre le pourcentage d'antagonisme de l'installation de la catalepsie provoquée par la prochlorpérazine.

Tableau II

| Composé | Dose | % d'antagonisme |
| --- | --- | --- |
| 2-(1-pipérazinyl)pyrimidine monomaléate | 0,1 mg/kg | 20 % |
| | 1 mg/kg | 80 % |

De ce tableau, il ressort que le monomaléate de 2-(1-pipérazinyl)pyrimidine de la présente invention est actif dans le test de la catalepsie provoquée par la prochlorpérazine, prédictif d'une activité antidépressive.

L'activité tranquillisante-sédative des composés de l'invention a été déterminée dans le test de la motilité des souris selon la technique de J. R. Boissier et al. (Arch. Int. Pharmacodyn. 1965, 158, 212).

Un composé représentatif de l'invention, à savoir le monomaléate de 2-(1-pipérazinyl)pyrimidine, a été administré par voie intrapéritonéale à des lots de 12 souris par dose ; en même temps, un lot d'animaux témoins n'a reçu que du sérum physiologique. Les animaux ont été placés individuellement 45 min après l'administration dans des cages actimétriques type Apelab (L = 26 cm, 1 = 21,5 cm, H = 10 cm) traversées par deux rayons lumineux qui impressionnent une cellule photo-électrique. Chaque traversée d'un faisceau lumineux a été comptabilisée par un compteur individuel. Les scores correspondant aux déplacements des animaux ont été enregistrés pendant 10 min.

Le tableau III ci-dessous montre la variation pour cent de la moitié spontanée des animaux traités aux différentes doses par rapport aux animaux témoins.

(Voir Tableau 3 p. 4)

Tableau III

| Composé | Dose | Variation % |
|---|---|---|
| 2-(1-pipérazinyl)pyrimidine monomaléate | 2,5 mg/kg | - 51 % ** |
| | 1,25 mg/kg | - 39 % * |
| | 0,60 mg/kg | - 24 % ns |

** p <0,01 * p <0,05 Test "t" de Student

De ce tableau, il ressort que le monomaléate de 2-(1-pipérazinyl)pyrimidine de la présente invention manifeste une bonne activité sédative enregistrée par une baisse de la motilité spontanée de la souris.

Le mécanisme dopaminergique d'un composé représentatif de l'invention, la 2-(1-pipérazinyl)pyrimidine mono-L-(+)-tartrate, a été étudié en comparaison avec la base libre correspondante et son chlorhydrate par l'analyse du comportement de rotation chez la souris après lésion unilatérale du striatum (P. Protais et al. J. Pharmacol. 1976, 7, 251).

Des souris femelles Charles River CD1 pesant de 20 à 24 g ont préalablement fait l'objet d'une lésion unilatérale du striatum par injection stéréotaxique de 6-hydroxydopamine à raison de 8 mcg par animal. Une semaine après cette opération, la 2-(1-pipérazinyl)pyrimidine mono-L-(+)-tartrate, la 2-(1-pipérazinyl)pyrimidine chlorhydrate et la base libre ont été administrées par voie orale à des groupes de 6 souris à une dose correspondant à 0,15 mg/kg de base libre. Le nombre de rotations a été évalué, pendant 2 minutes, 1, 3 et 6 heures après l'administration des trois produits. Les rotations ipsilatérales à la lésion ont été comptées positivement, celles contralatérales ont été comptées négativement. La somme algébrique des rotations pour chaque groupe d'animaux traités a été comparée à celle du groupe d'animaux témoins n'ayant reçu que le véhicule (sérum physiologique).

Le tableau IV ci-dessous montre la variation pour cent, par rapport aux animaux témoins, des rotations.

Tableau IV

| Composé | Variation % après minutes: | | |
|---|---|---|---|
| | 60 | 180 | 360 |
| 2-(1-pipérazinyl)-pyrimidine base | - 94 ** | - 62 ** | - 60 ** |
| 2-(1-pipérazinyl)-pyrimidine chlorhydrate | - 29 ns | - 27 ns | - 22 ns |
| 2-(1-pipérazinyl)-pyrimidine mono-L(+)-tartrate | - 88 ** | - 98 ** | -124 ** |

** significatif p < 0,01 Test "t" de Student
ns: non significatif

De ce tableau il ressort que la base réduit d'une façon significative la somme algébrique des rotations ipsilatérales et contralatérales à la lésion, alors que le chlorhydrate n'a pas d'effet significatif.

Ce tableau montre aussi que le chlorhydrate est inactif aussi dans le temps et que la base libre montre une tendance à la diminution de l'activité dans le temps. Par contre, la 2-(1-pipérazinyl)pyrimidine mono-L-(+)-tartrate de la présente invention est plus puissante dans le temps que les produits de référence, et,

en plus, son activité augmente significativement dans le temps. Plus particulièrement, le composé représentatif de l'invention, à la 6e heure, non seulement réduit la somme algébrique ci-dessus, mais il montre une variation négative supérieure à 100 %. Ce résultat surprenant prouve que le nombre des rotations contralatérales à la lésion est plus grande que le nombre des rotations ipsilatérales à la lésion.

L'activité psychotrope dopaminergique des composés de la présente invention et leur faible toxicité les rendent utiles comme médicaments.

Ainsi, selon un autre de ses aspects, la présente invention concerne des compositions pharmaceutiques renfermant les sels acides de la 2-(1-pipérazinyl)pyrimidine avec les acides dicarboxyliques en tant qu'ingrédients actifs.

Ingrédients actifs préférés sont les composés de formule I ci-dessus.

Dans les compositions pharmaceutiques de la présente invention pour l'administration orale, parentérale, sublinguale, transdermique ou rectale, le sel de la 2-(1-pipérazinyl)pyrimidine avec un acide dicarboxylique utilisé comme ingrédient actif, peut être administré sous formes unitaires d'administration, en mélange avec des supports pharmaceutiques classiques, aux animaux et aux êtres humains pour le traitement des troubles de l'humeur ou du comportement, notamment dans le cas de psychose, dépression, ainsi que des états anxieux et des insomnies. Parmi les formes unitaires d'administration appropriées, il y a les formes par voie orale telles que les comprimés, les gélules, les poudres, les granules et les solutions ou suspensions orales et les suppositoires pour une administration rectale.

Afin d'obtenir l'effet psychotrope désiré, la dose de principe actif peut varier entre 0,1 et 100 mg par kg de poids du corps et par jour.

Chaque dose unitaire peut contenir de 1 à 300 mg d'ingrédient actif en combinaison avec un support pharmaceutique. Cette dose unitaire peut être administrée 1 à 4 fois par jour pour le traitement des troubles de l'humeur ou du comportement.

Les exemples suivants illustrent l'invention sans toutefois la limiter.

### Exemple 1

On chauffe au reflux pendant 18 heures un mélange de 3,2 g de 2-chloropyrimidine et de 12,1 g de pipérazine anhydre dans 100 ml d'éthanol absolu, puis on évapore à fond sous pression réduite et à chaud. On reprend le résidu avec 400 ml d'éther diéthylique, on lave la solution ainsi obtenue d'abord avec 10 ml d'une solution aqueuse d'hydroxyde de sodium 1 : 1, puis avec 20 ml d'eau. On sèche la solution organique sur du sulfate de sodium anhydre, on la filtre et on l'évapore à sec sous pression réduite. On dissout l'huile ainsi obtenue dans 30 ml d'isopropanol et on ajoute à la solution 2,7 g d'acide maléique dissous dans 30 ml d'isopropanol à 60 °C environ. De la solution ainsi obtenue cristallise le produit qui est filtré et séché. On obtient 4 g de monomaléate de 2-(1-pipérazinyl)pyrimidine ; F. 160-162 °C. Par cristallisation ultérieure de l'éthanol à 95 %, le point de fusion ne change pas.

### Exemple 2

A une solution de 1,64 g de 2-(1-pipérazinyl)pyrimidine dans 15 ml d'isopropanol, on ajoute, à la température de 50-60 °C, une solution de 1,16 g d'acide maléique dans 15 ml d'isopropanol. On obtient ainsi le monomaléate de 2-(1-pipérazinyl)pyrimidine, identique au produit de l'Exemple 1.

De la même façon, on obtient :
le monofumarate de 2-(1-pipérazinyl)pyrimidine, F. 201-203 °C,
le mono-L(+)-tartrate de 2-(1-pipérazinyl)pyrimidine, F. 165-168 °C,
le monosuccinate de 2-(1-pipérazinyl)pyrmidine, F. 173-176 °C.

Le rendement dans toutes ces préparations est d'environ 95 %.

### Exemple 3

A une solution de 1,64 g de 2-(1-pipérazinyl)pyrimidine dans 100 ml d'acétone, on ajoute, à la température de 45 °C, une solution aqueuse de 1,47 g d'acide glutamique. On évapore à sec et on reprend le résidu par de l'éther de pétrole. On obtient ainsi le mono-L-glutamate de 2-(1-pipérazinyl)pyrimidine ; F. 188-190 °C ; rendement 95 %.

### Exemple 4

On prépare des gélules à base d'un des composés des exemples 1 à 3 ayant la composition suivante :

| | |
|---|---|
| principe actif | 15 mg |
| lactose | 120 mg |
| stéarate de magnésium | 5 mg |

en mélangeant intimement des charges des ingrédients ci-dessus et en versant le mélange dans des gélules de gélatine dure.

Exemple 5

On prépare des comprimés à base d'un des composés des exemples 1 à 3 ayant la composition suivante :

| | |
|---|---|
| principe actif | 20 mg |
| lactose | 100 mg |
| cellulose microcristalline | 30 mg |
| amidon de maïs séché | 40 mg |
| stéarate de magnésium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

Exemple 6

En opérant comme décrit dans l'Exemple 5 ci-dessus, on prépare des comprimés ayant la composition suivante :

| | |
|---|---|
| principe actif | 50 mg |
| lactose | 95 mg |
| amidon de maïs | 100 mg |
| talc | 4,5 mg |
| stéarate de magnésium | 0,5 mg |

Exemple 7

On prépare des suppositoires à base d'un des composés des exemples 1 à 3 ayant la composition suivante :

| | |
|---|---|
| principe actif | 50 mg |
| lactose | 250 mg |
| masse pour suppositoires | q.s.p. 1,7 g |

On mélange la substance active avec le lactose et on met le mélange uniformément en suspension dans la masse pour suppositoires fondue. On verse la suspension dans des moules refroidis pour former des suppositoires d'un poids de 1,7 g.

**Revendications** (pour les Etats contractants : BE, CH, DE, FR, GB, IT, LI, LU, NL, SE)

1. Un sel acide de la 2-(1-pipérazinyl)pyrimidine avec un acide dicarboxylique.
2. Un sel selon la revendication 1, ayant la formule

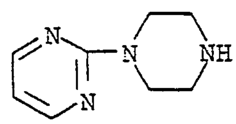 , HOOC-X-COOH

dans laquelle X représente un groupe alkylène ayant 1 à 3 atomes de carbone, un groupe vinylène, un groupe hydroxyéthylène, un groupe 1,2-dihydroxyéthylène, un groupe aminoéthylène ou un groupe amino-1,3-propylène.
3. Monomaléate de 2-(1-pipérazinyl)pyrimidine.
4. Monofuramate de 2-(1-pipérazinyl)pyrimidine.
3. Monosuccinate de 2-(1-pipérazinyl)pyrimidine.
6. Mono-L-(+)-tartrate de 2-(1-pipérazinyl)pyrimidine.
7. Procédé pour la préparation de sels acides de la 2-(1-pipérazinyl)pyrimidine avec des acides dicarboxyliques caractérisé en ce que l'on traite la 2-(1-pipérazinyl)pyrimidine avec la quantité calculée d'un acide dicarboxylique dans un solvant organique ou organique aqueux.
8. Procédé selon la revendication 7 caractérisé en ce que la salification est conduite sur une 2-(1-pipérazinyl)pyrimidine brute, telle qu'obtenue de la réaction entre la 2-chloropyrimidine et la pipérazine.
9. Procédé selon la revendication 7 caractérisé en ce que comme acide dicarboxylique on utilise un composé de formule

HOOC—X—COOH

dans laquelle X est tel que défini dans la revendication 2.

10. Procédé selon la revendication 9, caractérisé en ce que l'acide dicarboxylique utilisé est en quantité équimoléculaire.

11. Composition pharmaceutique à action psychotrope dopaminergique renfermant, en tant qu'ingrédient actif, un composé selon une des revendications 1 à 6.

12. Composition pharmaceutique selon la revendication 11, sous forme de dosage unitaire.

13. Composition pharmaceutique selon la revendication 12, caractérisée en ce qu'elle contient, pour chaque unité de dosage, de 1 à 300 mg d'ingrédient actif en mélange avec un excipient pharmaceutique.

**Revendications** (pour l'Etat contractant AT)

1. Procédé pour la préparation de sels acides de la 2-(1-pipérazinyl)pyrimidine avec des acides dicarboxyliques, caractérisé en ce que l'on traite la 2-(1-pipérazinyl)pyrimidine avec la quantité calculée d'un acide dicarboxylique dans un solvant organique ou organique aqueux.

2. Procédé selon la revendication 1, caractérisé en ce que le sel acide de la 2-(1-pipérazinyl) pyrimidine répond à la formule

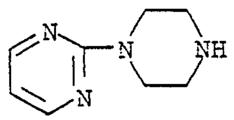 , HOOC-X-COOH

dans laquelle X représente un groupe alkylène ayant 1 à 3 atomes de carbone, un groupe vinylène, un groupe hydroxyéthylène, un groupe 1,2-dihydroxyéthylène, un groupe aminoéthylène ou un groupe amino-1,3-propylène.

3. Procédé selon l'une des revendications 1 ou 2, caractérisé en ce que la salification est conduite sur une 2-(1-pipérazinyl)pyrimidine brute, telle qu'obtenue de la réaction entre la 2-chloropyrimidine et la pipérazine.

4. Procédé selon l'une quelconque des revendications 1 à 3, caractérisé en ce que comme acide dicarboxylique on utilise un composé de formule

HOOC—X—COOH

dans laquelle X est tel que défini dans la revendication 2.

5. Procédé selon l'une quelconque des revendications 1 à 4, caractérisé en ce que l'acide dicarboxylique utilisé est en quantité équimoléculaire.

6. Procédé selon l'une quelconque des revendications 1 à 5, caractérisé en ce que le sel d'acide est le monomaléate de 2-(1-pipérazinyl)pyrimidine.

7. Procédé selon l'une quelconque des revendications 1 à 5, caractérisé en ce que le sel d'acide est le monofumarate de 2-(1-pipérazinyl)pyrimidine.

8. Procédé selon l'une quelconque des revendications 1 à 5, caractérisé en ce que le sel d'acide est le monosuccinate de 2-(1-pipérazinyl)pyrimidine.

9. Procédé selon l'une quelconque des revendications 1 à 5, caractérisé en ce que le sel est le mono-L(+)-tartrate de 2-(1-pipérazinyl)pyrimidine.

10. Utilisation d'un sel acide de la 2-(1-pipérazinyl)pyrimidine avec un sel acide dicarboxylique obtenu par le procédé selon l'une quelconque des revendications 1 à 9, pour la préparation d'une composition pharmaceutique à action psychotrope dopaminergique contenant un tel sel à titre d'ingrédient actif.

11. Utilisation selon la revendication 10, caractérisée en ce que la composition est sous forme de dosage unitaire et en ce qu'elle contient pour chaque unité de dosage, de 1 à 300 mg d'ingrédient actif en mélange avec un excipient pharmaceutique.

**Claims** (for the Contracting States : BE, CH, DE, FR, GB, IT, LI, LU, NL, SE)

1. An acid addition salt of 2-(1-piperazinyl)pyrimidine with a dicarboxylic acid.

2. A salt according to claim 1, of formula

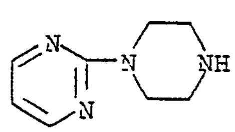 , HOOC-X-COOH

wherein X represents an alkylene group of from 1 to 3 carbon atoms, a vinylene group, a hydroxyethylene group, a 1,2-dihydroxyethylene group, an aminoethylene group or an amino-1,3-propylene group.

3. 2-(1-piperazinyl)pyrimidine monomaleate.

4. 2-(1-piperazinyl)pyrimidine monofuramate.

5. 2-(1-piperazinyl)pyrimidine monosuccinate.

6. 2-(1-piperazinyl)pyrimidine mono-L(+) tartrate.

7. A process for the preparation of acid addition salts of 2-(1-piperazinyl)pyrimidine with dicarboxylic acids, characterized in that it comprises reacting 2-(1-piperazinyl)pyrimidine with a calculated amount of a dicarboxylic acid in an organic or organic aqueous solvent.

8. A process according to claim 7, characterized in that salification is carried out on a crude 2-(1-piperazinyl)pyrimidine, as obtained from the reaction of 2-chloropyrimidine with piperazine.

9. A process according to claim 7, characterized in that the dicarboxylic acid utilized is a compound of formula

HOOC—X—COOH

wherein X is as defined in claim 2.

10. A process according to claim 9, characterized in that the dicarboxylic acid is utilized in equimolecular amount.

11. A pharmaceutical composition having dopaminergic psychotropic action containing, as active ingredient, a compound according to any one of claims 1 to 6.

12. A pharmaceutical composition according to claim 11, which is in dosage unit form.

13. A pharmaceutical composition according to claim 12, characterized in that it contains from 1 to 300 mg of active ingredient in admixture with a pharmaceutical carrier per dosage unit.

**Claims** (for the contracting state : AT)

1. A process for preparing acid salts of 2-(1-piperazinyl)pyrimidine with dicarboxylic acids, characterized in that it comprises reacting 2-(1-piperazinyl)-pyrimidine with the calculated quantity of a dicarboxylic acid in an organic or aqueous organic solvent.

2. Process according to claim 1, characterized in that the acid salt of 2-(1-piperazinyl)pyrimidine responds to the formula

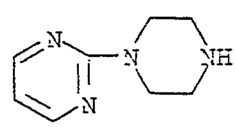

, HOOC-X-COOH

wherein X represents an alkylene group of from 1 to 3 carbon atoms, a vinylene group, a hydroxyethylene group, a 1,2-dihydroxyethylene group, an aminoethylene group or an amino-1,3-propylene group.

3. Process according to any one of claims 1 or 2, characterized in that salification is carried out on a crude 2-(1-piperazinyl)pyrimidine, as obtained from the reaction of 2-chloropyrimidine with piperazine.

4. A process according to any one of claims 1 to 3, characterized in that the dicarboxylic acid utilized is a compound of formula.

HOOC—X—COOH

wherein X is as claimed in claim 2.

5. Process according to any one of claims 1 to 4, characterized in that the dicarboxylic acid is utilized in equimolecular amount.

6. Process according to any one of claims 1 to 4, characterized in that the acid salt is 2-(1-piperazinyl)pyrimidine monomaleate.

7. Process according to any one of claims 1 to 5, characterized in that the acid salt is 2-piperazinyl)pyrimidine monofumarate.

8. Process according to any one of claims 1 to 5, characterized in that the acid salt is 2-(1-piperazinyl)pyrimidine monosuccinate.

9. Process according to any one of claims 1 to 5, characterized in that the salt is 2-(1-piperazinyl) pyrimidine mono-L(+)tartrate.

10. Use of an acid salt of 2-(1-piperazinyl)pyrimidine with an acid dicarboxylic salt obtained by the process according to any one of claims 1 to 9, for the preparation of a pharmaceutical composition having dopaminergic psychotropic action containing such a salt as active ingredient.

11. Use according to claim 10, characterized in that the composition is in dosage unit form and in that it contains from 1 to 300 mg of active ingredient in admixture with a pharmaceutical carrier per dosage unit.

**Patentansprüche** (für die Vertragsstaaten : BE, CH, DE, FR, GB, IT, LI, LU, NL, SE)

1. Saures Salz von 2-(Piperazin-1-yl)-pyrimidin mit einer Dicarbonsäure.

2. Salz nach Anspruch 1 der Formel

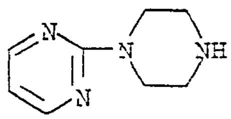 HOOC-X-COOH

worin X eine Alkylengruppe mit 1 bis 3 Kohlenstoffatomen, eine Vinylengruppe, eine Hydroxyäthylengruppe, eine 1,2-Dihydroxyäthylengruppe, eine Aminoäthylengruppe oder eine Amino-1,3-propylengruppe darstellt.

3. Monomaleat von 2-(Piperazin-1-yl)-pyrimidin.

4. Monofumarat von 2-(Piperazin-1-yl)-pyrimidin.

5. Monosuccinat von 2-(Piperazin-1-yl)-pyrimidin.

6. Mono-L(+)-tartrat von 2-(Piperazin-1-yl)-pyrimidin.

7. Verfahren zur Herstellung von sauren Salzen von 2-(Piperazin-1-yl)-pyrimidin mit Dicarbonsäure, dadurch gekennzeichnet, daß man 2-(Piperazin-1-yl)-pyrimidin mit der errechneten Menge einer Dicarbonsäure in einem organischen oder wässerig-organischen Lösungsmittel behandelt.

8. Verfahren nach Anspruch 7, dadurch gekennzeichnet, daß die Salzbildung an einem rohen 2-(Piperazin-1-yl)-pyrimidin, wie es bei der Umsetzung von 2-Chlorpyrimidin mit Piperazin erhalten wird, durchgeführt wird.

9. Verfahren nach Anspruch 7, dadurch gekennzeichnet, daß man als Dicarbonsäure eine Verbindung der Formel

HOOC—X—COOH

verwendet, worin X die in Anspruch 2 angegebene Bedeutung hat.

10. Verfahren nach Anspruch 9, dadurch gekennzeichnet, daß die Dicarbonsäure in äquimolarer Menge verwendet wird.

11. Pharmazeutische Zusammensetzung mit psychotroper dopaminergischer Wirkung, die als Wirkstoff eine Verbindung nach einem der Ansprüche 1 bis 6 enthält.

12. Pharmazeutische Zusammensetzung nach Anspruch 11 in Dosiseinheitsform.

13. Pharmazeutische Zusammensetzung nach Anspruch 12, dadurch gekennzeichnet, daß sie pro Dosiseinheit 1 bis 300 mg aktives Ingrediens in Mischung mit einem pharmazeutischen Exzipienten enthält.

**Patentansprüche** (für den Vertragsstaat AT)

1. Verfahren zur Herstellung von sauren Salzen von 2-(Piperazin-1-yl)-pyrimidin mit Dicarbonsäuren, dadurch gekennzeichnet, daß man 2-(Piperazin-1-yl)-pyrimidin mit der errechneten Menge einer Dicarbonsäure in einem organischen oder wässerig-organischen Lösungsmittel behandelt.

2. Verfahren nach Anspruch 1, dadurch gekennzeichnet, daß das saure Salz von 2-(Piperazin-1-yl)-pyrimidin die folgende Formel hat :

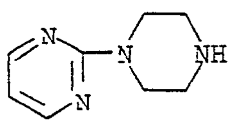 , HOOC-X-COOH

worin X eine Alkylengruppe mit 1 bis 3 Kohlenstoffatomen, eine Vinylengruppe, eine Hydroxyäthylengruppe, eine 1,2-Dihydroxyäthylengruppe, eine Aminoäthylengruppe oder eine Amino-1,3-propylengruppe darstellt.

3. Verfahren nach einem der Ansprüche 1 oder 2, dadurch gekennzeichnet, daß die Salzbildung an einem rohen 2-(Piperazin-1-yl)-pyrimidin, wie es bei der Umsetzung von 2-Chlorpyrimidin mit Piperazin erhalten wird, durchgeführt wird.

4. Verfahren nach einem der Ansprüche 1 bis 3, dadurch gekennzeichnet, daß man als Dicarbonsäure eine Verbindung der Formel

HOOC—X—COOH

verwendet, worin X die in Anspruch 2 angegebene Bedeutung hat.

5. Verfahren nach einem der Ansprüche 1 bis 4, dadurch gekennzeichnet, daß die Dicarbonsäure in äquimolarer Menge verwendet wird.

6. Verfahren nach einem der Ansprüche 1 bis 5, dadurch gekennzeichnet, daß das saure Salz 2-(Piperazin-1-yl)-pyrimidin-monomaleat ist.

7. Verfahren nach einem der Ansprüche 1 bis 5, dadurch gekennzeichnet, daß das saure Salz 2-(Piperazin-1-yl)-pyrimidin-monofumarat ist.

8. Verfahren nach einem der Ansprüche 1 bis 5, dadurch gekennzeichnet, daß das saure Salz 2-(Piperazin-1-yl)-pyrimidin-monosuccinat ist.

9. Verfahren nach einem der Ansprüche 1 bis 5, dadurch gekennzeichnet, daß das Salz Mono-L(+)-tartrat von 2-(Piperazin-1-yl)-pyrimidin ist.

10. Verwendung eines sauren Salzes von 2-(Piperazin-1-yl)-pyrimidin mit einer Dicarbonsäure (salz), erhalten durch das Verfahren nach einem der Ansprüche 1 bis 9, zur Herstellung einer pharmazeutischen Zusammensetzung mit psychotroper, dopaminergischer Wirkung, die als Wirkstoff ein solches Salz enthält.

11. Verwendung nach Anspruch 10, dadurch gekennzeichnet, daß die Zusammensetzung in Dosiseinheitsform vorliegt und daß sie pro Dosiseinheit 1 bis 300 mg aktives Ingrediens in Mischung mit einem pharmazeutischen Exzipienten enthält.